# EUROPEAN PATENT SPECIFICATION

(11) **EP 4 572 713 B1**
(45) Date of publication and mention of the grant of the patent: **04.02.2026**
(21) Application number: 23768708.2
(22) Date of filing: 18.08.2023
(51) Int. Cl.: A61F 2/24

(54) **PROSTHETIC HEART VALVE**
HERZKLAPPENPROTHESE
VALVULE CARDIAQUE PROTHÉTIQUE

(30) Priority: 19.08.2022 US 202263399604 P; 19.08.2022 US 202263399626 P
(43) Date of publication of application: 25.06.2025
(73) Proprietor: Edwards Lifesciences Corporation, Irvine, CA 92614 (US)
(72) Inventor: GUROVICH, Nikolai, 3079892 Caesarea (IL); BUKIN, Michael, 3079892 Caesarea (IL); NGUYEN, Tammy, Irvine, CA 92614 (US); PHAM, Bich Hoang, Irvine, CA 92614 (US); HAN, Jiangxue, Irvine, CA 92614 (US)
(74) Representative: Eisenführ Speiser
(86) International application number: PCT/US2023/030530
(87) International publication number: WO 2024/039817

(56) References cited:
- WO-A1-2022/016066
- WO-A1-2022/103747
- US-A1- 2020 138 573

## Description

### CROSS-REFERENCE TO RELATED APPLICATIONS

This application claims the benefit of U.S. Provisional Application Nos. 63/399,604, filed August 19, 2022, and 63/399,626, filed August 19, 2022,

### FIELD

The present disclosure relates to prosthetic heart valves, and in particular to leaflets for prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

Most expandable, prosthetic heart valves comprise a cylindrical metal frame or stent and prosthetic leaflets mounted inside the frame. Each leaflet can comprise a main body with a cusp edge portion and one or more sets of commissure tabs extending from the main body on opposite sides of the leaflet. The leaflets can be secured to one another at adjacent commissure tabs to form commissures that are then secured to commissure windows in the frame of the prosthetic heart valve. The cusp edge portion of each leaflet can also be secured to struts of the frame. The leaflets of the prosthetic heart valve are configured to open and close to regulate a flow of blood through the prosthetic heart valve, from an inflow end to an outflow end of the prosthetic heart valve.

WO 2022/103747 A1 discloses a leaflet for a valvular structure of a prosthetic heart valve that comprises a first portion, a second portion, and first and second tabs. The first and second tabs are on opposite sides of the first portion with respect to a centerline of the first portion. Each of the tabs has a base edge and an outer edge. The outer edges of the first and second tabs are substantially parallel to each other. The second portion has a half-elliptical or semi-elliptical shape that defines a cusp edge. The cusp edge extends from the base edge of the first tab to the base edge of the second tab. The cusp edge is curved along its entire length between the base edges of the first and second tabs.

### SUMMARY

Described herein are prosthetic heart valves, delivery apparatus, and methods for implanting prosthetic heart valves. Also described herein are leaflets configured to be mounted inside a frame of a prosthetic heart valve, and methods for assembling the leaflets together into a leaflet assembly and attaching the leaflets to the frame. The disclosed leaflets, prosthetic heart valves, and methods can, for example, provide more durable leaflets that also open wider during operation of the prosthetic heat valve, thereby decreasing a pressure gradient across the prosthetic valve. As such, the devices and methods disclosed herein can, among other things, overcome one or more of the deficiencies of typical prosthetic heart valves.

A leaflet for a prosthetic valve can comprise a main body with a free, outflow edge and a cusp edge portion, two lower tabs disposed on opposite sides of the main body, and two upper tabs disposed on opposite sides of the main body.

In some examples, a leaflet can comprise two offsetting portions, each offsetting portion extending between a respective lower tab and upper tab and offsetting the respective upper tab axially and laterally away from the outflow edge of the main body, wherein each upper tab has opposing inner and outer edges that are parallel to one another and disposed parallel to the central longitudinal axis of the leaflet.

In some examples, a leaflet can comprise two offsetting portions, each offsetting portion extending between a respective lower tab and upper tab and offsetting the respective upper tab axially and laterally away from the free edge of the main body. A first width of each upper tab of the two upper tabs is wider than a second width of each lower tab of the two lower tabs such that an outer edge of each upper tab extends laterally outward farther than an outer edge of a respective lower tab, relative to the central longitudinal axis of the leaflet.

In some examples, a leaflet can comprise two offsetting portions, each offsetting portion extending between a respective lower tab and upper tab and offsetting the respective upper tab axially and laterally away from the free edge of the main body. Each offsetting portion has an outer edge that extends between an inflow edge of the respective upper tab and an outflow edge of the respective lower tab and an arcuate inner edge that curves between an inner edge of the respective upper tab and the free edge of the main body.

In some examples, a leaflet for a prosthetic valve comprises a main body with a free, outflow edge and a cusp edge portion; two lower tabs disposed on opposite sides of the main body, wherein the cusp edge portion terminates at upper ends thereof at the lower tabs, and the lower tabs extend laterally outward from the main body relative to a central longitudinal axis of the leaflet; two upper tabs disposed on opposite sides of the main body and extending laterally outward from the main body; and two offsetting portions, each offsetting portion extending between a respective lower tab and upper tab and offsetting the respective upper tab axially and laterally away from the outflow edge of the main body, wherein each upper tab has opposing inner and outer edges that are parallel to one another and disposed parallel to the central longitudinal axis of the leaflet.

In some examples, a leaflet for a prosthetic valve comprises a main body with a free edge and a cusp edge portion, the free edge disposed at an outflow end of the leaflet; two lower tabs disposed on opposite sides of the main body, wherein the cusp edge portion terminates at upper ends thereof at the lower tabs, and the lower tabs extend laterally outward from the main body relative to a central longitudinal axis of the leaflet; two upper tabs disposed on opposite sides of the main body and extending laterally outward from the main body; and two offsetting portions, each offsetting portion extending between a respective lower tab and upper tab and offsetting the respective upper tab axially and laterally away from the free edge of the main body, wherein a first width of each upper tab of the two upper tabs is wider than a second width of each lower tab of the two lower tabs such that an outer edge of each upper tab extends laterally outward farther than an outer edge of a respective lower tab, relative to the central longitudinal axis of the leaflet, and wherein the first and second widths extend perpendicular to the central longitudinal axis of the leaflet.

In some examples, a leaflet for a prosthetic valve comprises a main body with a free edge disposed at its outflow end and a cusp edge portion defining its inflow end; two lower tabs disposed on opposite sides of the main body, wherein the cusp edge portion terminates at upper ends thereof at the lower tabs, and the lower tabs extend laterally outward from the main body relative to a central longitudinal axis of the leaflet; two upper tabs disposed on opposite sides of the main body and extending laterally outward from the main body; and two offsetting portions, each offsetting portion extending between a respective lower tab and upper tab and offsetting the respective upper tab axially and laterally away from the free edge of the main body, wherein each offsetting portion has an outer edge that extends between an inflow edge of the respective upper tab and an outflow edge of the respective lower tab and an arcuate inner edge that curves between an inner edge of the respective upper tab and the free edge of the main body, wherein the inner edge of each offsetting portion is disposed closer to the central longitudinal axis than the outer edge of the offsetting portion.

In some examples, a leaflet for a prosthetic valve comprises one or more of the components recited in Examples 1-9, 12-19, and 22-30 below.

A prosthetic heart valve can comprise a frame and a valve structure coupled to the frame. In addition to these components, a prosthetic heart valve can further comprise one or more of the components disclosed herein.

In some examples, a prosthetic heart valve can comprise a sealing member configured to reduce paravalvular leakage.

In some examples, a prosthetic heart valve comprises a frame that is radially expandable and collapsible between a radially expanded and radially collapsed configuration, where the frame comprises a plurality of interconnected struts including a plurality of rows of angled struts and a plurality of axially extending window strut portions defining a plurality of circumferentially spaced apart commissure windows. The prosthetic heart valve further comprises a valvular structure mounted on an inside of the frame and comprising a plurality of leaflets, where each leaflet comprises a main body with a free, outflow edge and a cusp edge portion, a pair of lower tabs disposed on opposite sides of the main body, and a pair of upper tabs disposed on opposite sides of the main body, where the pairs of lower and upper tabs of adjacent leaflets are paired to form a commissure that is secured to a respective commissure window of the frame. The prosthetic heart valve further comprises an inner skirt disposed around an inner surface of the frame and secured to the cusp edge portion of each leaflet, where the inner skirt is attached to a first row of angled struts forming an inflow end of the frame and a second row of angled struts disposed adjacent to an outflow end of the frame. The prosthetic heart valve further comprises an outer skirt disposed around an outer surface of the frame and attached to the first row of angled struts and a third row of angled struts, where the inner skirt and the outer skirt are attached together with a single line of stitches to a fourth row of angled struts that is disposed between the second row of angled struts and the third row of angled struts, and where a single knot tail for the single line of stitches is formed around an angled strut of the fourth row of angle struts that is disposed below one of the commissure windows.

In some examples, a prosthetic heart valve comprises one or more of the components recited in Examples 10, 11, 20, 21, 31, 32-36, and 38 below.

This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a perspective view of a prosthetic heart valve.
FIG. 2 is a perspective view of a delivery apparatus for a prosthetic heart valve, according to an example.
FIG. 3 is a plan view of a leaflet for a prosthetic heart valve.
FIG. 4 is schematic of a portion of the leaflet of FIG. 3, showing an upper tab of the leaflet folded over a lower tab of the leaflet.
FIG. 5 is a perspective view of a prosthetic valve including a frame and a valvular structure comprising a plurality of the leaflets of FIG. 3 mounted on an inside of the frame.
FIG. 6 is a detail view of a portion of the prosthetic valve of FIG. 5 showing a commissure of the prosthetic valve.
FIG. 7A is a top view of the prosthetic valve of FIG. 5.
FIG. 7B is a top view of the prosthetic valve of FIG. 5 when the valve is in a fully open position during operation of the valve.
FIG. 8 is a top perspective view of a portion of the prosthetic valve of FIG. 5 showing a commissure of the prosthetic valve attached to a commissure window of the frame.
FIGS. 9-12B show the assembly of commissures of the valvular structure to commissure windows of the frame of the prosthetic valve.
FIG. 13A-15 show the assembly of an inner and outer skirt to the frame of the prosthetic valve.
FIG. 16 is a graph of pressure gradient across two different prosthetic valves having a first size, during steady forward flow through the valves, where one of the valves comprises a plurality of the leaflets of FIG. 3.
FIG. 17 is a graph of pressure gradient across two different prosthetic valves having a second size, during steady forward flow through the valves, where one of the valves comprises a plurality of the leaflets of FIG. 3.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

As used herein, "e.g." means "for example," and "i.e." means "that is."

### Overview of the Disclosed Technology

As introduced above, a leaflet assembly comprising a plurality of leaflets can be mounted on an inside of a frame of a prosthetic heart valve. The leaflet assembly is configured to regulate blood flow through the prosthetic heart valve, from an inflow end to an outflow end of the prosthetic heart valve. Each leaflet can comprise a main body with a cusp edge portion and two sets of commissure tabs extending from the main body on opposite sides of the leaflet. The two sets of commissure tabs can comprise a pair of opposing upper tabs and a pair of opposing lower tabs. The leaflets can be secured to one another at adjacent commissure tabs to form commissures that are then secured to commissure windows in the frame of the prosthetic heart valve. The cusp edge portion of each leaflet can also be secured to struts of the frame. Thus, the main body of each leaflet, between a free (or outflow) edge of the leaflet and the cusp edge portion can be referred to as a "movable" portion of the leaflet that opens and closes during operation of the prosthetic heart valve (during systole and diastole).

Some leaflet designs with shorter (in width) upper tabs and/or upper tabs with an angled inner edge that connects to the free edge of the leaflet can result in a leaflet assembly that does not open wide enough (e.g., toward the frame) and/or can result in contact between the angled inner edge of the upper tabs and the movable portions of the leaflets, which can result in higher pressure gradients across the valve and reduced long-term durability of the leaflets.

Disclosed herein is a leaflet that includes upper tabs that are offset from the free edge of the leaflet by a relatively narrow offsetting portion. The upper tabs have an inner edge that is relatively straight and parallel to a central longitudinal axis of the leaflet (the central longitudinal axis extending between the outflow end and the inflow end of the leaflet). In some examples, the offsetting portion is curved and offsets a lower, inner corner of the upper tabs both axially and laterally away from the free edge of the leaflet. The upper tabs can also be wider (in a lateral direction that extends in a direction of the free edge of the leaflet) and extend farther outward from the body of the leaflet than the lower tabs.

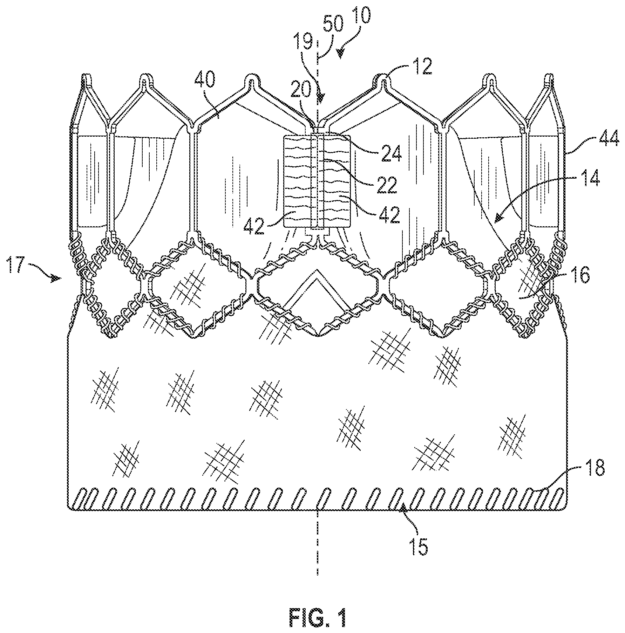

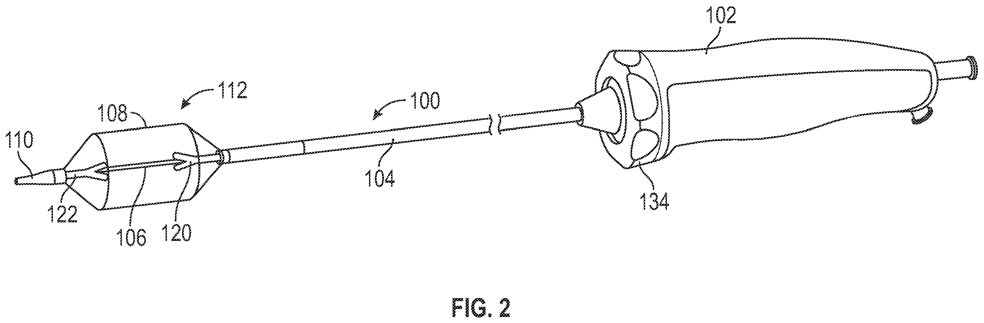

Prosthetic valves disclosed herein, such as the prosthetic heart valve shown in FIG. 1, can be radially compressible and expandable between a radially compressed state and a radially expanded state. Thus, the prosthetic valves can be crimped on or retained by an implant delivery apparatus in the radially compressed state while being advanced through a patient's vasculature on the delivery apparatus, such as the delivery apparatus shown in FIG. 2. The prosthetic valve can be expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

### Examples of the Disclosed Technology

FIG. 1 shows an exemplary prosthetic valve 10, according to one example. Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756, In some examples, the disclosed prosthetic valves can be implanted within a docking device implanted within or at the native mitral valve, such as disclosed in PCT Publication No. WO2020/247907, In some examples, the disclosed prosthetic valves can be implanted within a docking device implanted within the superior or inferior vena cava for replacing the function of a diseased tricuspid valve, such as disclosed in U.S. Publication No. 2019/0000615,

The prosthetic valve 10 comprises four main components: a stent or frame 12, a valvular structure 14, an inner skirt 16, and a perivalvular outer sealing member or outer skirt 18. The prosthetic valve 10 can have an inflow end portion 15 (also referred to herein as an "inflow end"), an intermediate portion 17, and an outflow end portion 19. The inner skirt 16 can be arranged on and/or coupled to an inner surface of the frame 12, while the outer skirt 18 can be arranged on and/or coupled to an outer surface of the frame 12.

The valvular structure 14 can comprise three leaflets 40, collectively forming a leaflet structure, which can be arranged to collapse in a tricuspid arrangement, although in some instances there can be greater or fewer number of leaflets (e.g., one or more leaflets 40). The leaflets 40 can be secured to one another at their adjacent sides to form commissures 22 of the valvular (e.g., leaflet) structure 14. The lower edge of valvular structure 14 can have an undulating, curved scalloped shape and can be secured to the inner skirt 16 by sutures (not shown). In some examples, the leaflets 40 can be formed of pericardial tissue (e.g., bovine pericardial tissue), biocompatible synthetic materials, or various other suitable natural or synthetic materials as known in the art and described in U.S. Patent No. 6,730,118,

The frame 12 can be formed with a plurality of circumferentially spaced slots, or commissure windows 20 that are adapted to mount the commissures 22 of the valvular structure 14 to the frame. For example, the commissure windows 20 can be defined by axially extending window strut portions 24 of the frame 12, which can also be referred to herein as "commissure supports". Each commissure window 20 is adapted to receive a pair of commissure tabs 42 of a pair of adjacent leaflets 40 arranged into a corresponding commissure 22. As shown in FIG. 1 and described further below, the pair of commissure tabs 42 extend from inside the frame 12, through the commissure window 20, and exterior to the frame 12. For example, as shown in FIG. 1, the commissure tabs 42 can extend over and/or protrude radially outward from an outer surface 44 (radially outward facing surface) of the frame 12, and in particular, outer surfaces of the window strut portions 24.

The frame 12 can be made of any of various suitable plastically-expandable materials (e.g., stainless steel, etc.) or self-expanding materials (e.g., Nitinol). When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frames disclosed herein (e.g., the frame 12) include, metal alloys, polymers, or combinations thereof. Example metal alloys can comprise one or more of the following: nickel, cobalt, chromium, molybdenum, titanium, or other biocompatible metal. In some examples, the frame 12 can comprise stainless steel. In some examples, the frame 12 can comprise cobalt-chromium. In some examples, the frame 12 can comprise nickel-cobalt-chromium. In some examples, the frame 12 comprises a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight.

FIG. 2 shows a delivery apparatus 100, according to an example, that can be used to implant an expandable prosthetic heart valve (e.g., prosthetic valve 10), or another type of expandable prosthetic medical device (such as a stent). In some examples, the delivery apparatus 100 is specifically adapted for use in introducing a prosthetic valve into a heart.

The delivery apparatus 100 in the illustrated example of FIG. 2 is a balloon catheter comprising a handle 102, a steerable, outer shaft 104 extending from the handle 102, an intermediate shaft extending from the handle 102 coaxially through the steerable outer shaft 104, and an inner shaft 106 extending from the handle 102 coaxially through the intermediate shaft and the steerable, outer shaft 104, an inflatable balloon (e.g., balloon) 108 extending from a distal end of the intermediate shaft, and a nosecone 110 arranged at a distal end of the delivery apparatus 100. A distal end portion 112 of the delivery apparatus 100 includes the balloon 108, the nosecone 110, and a balloon shoulder assembly. A prosthetic medical device, such as a prosthetic heart valve may be mounted on a valve retaining portion of the balloon 108. A balloon shoulder assembly is configured to maintain the prosthetic heart valve or other medical device at a fixed position on the balloon 108 during delivery through the patient's vasculature. In some examples, the balloon shoulder assembly can include a proximal shoulder 120 and/or a distal shoulder 122.

The balloon 108 can include a central portion (which can be approximately cylindrical when inflated, as shown in FIG. 2) and two tapered end portions that connect to the delivery apparatus 100 (e.g., to one or more shafts and/or a nosecone of the delivery apparatus).

The handle 102 can include a steering mechanism configured to adjust the curvature of the distal end portion of the delivery apparatus. In the illustrated example, for example, the handle 102 includes an adjustment member, such as the illustrated rotatable knob 134, which in turn is operatively coupled to the proximal end portion of a pull wire (not shown). The pull wire extends distally from the handle 102 through the outer shaft 104 and has a distal end portion affixed to the outer shaft at or near the distal end of the outer shaft 104. Rotating the knob 134 is effective to increase or decrease the tension in the pull wire, thereby adjusting the curvature of the distal end portion of the delivery apparatus.

The delivery apparatus 100 can be configured to be advanced over a guidewire that can be received within a guidewire lumen defined by an innermost shaft of the delivery apparatus 100.

In some examples, the delivery apparatus (or another, similar delivery apparatus) can be configured to deploy and implant a prosthetic heart valve (e.g., prosthetic valve 10 of FIG. 1) in the native aortic annulus of a native aortic valve. Further details on such a delivery apparatus can be found in International Application No. PCT/US2021/047056,

As an example, during an implantation procedure for implanting an expandable prosthetic heart valve (e.g., prosthetic valve 10 of FIG. 1), the distal end portion of the delivery apparatus 100 (or another similar delivery apparatus or balloon catheter) can be advanced (over a guidewire) to a target implantation site (e.g., a native valve annulus). The balloon 108 can then be inflated to radially expand and implant the prosthetic heart valve within the native valve annulus.

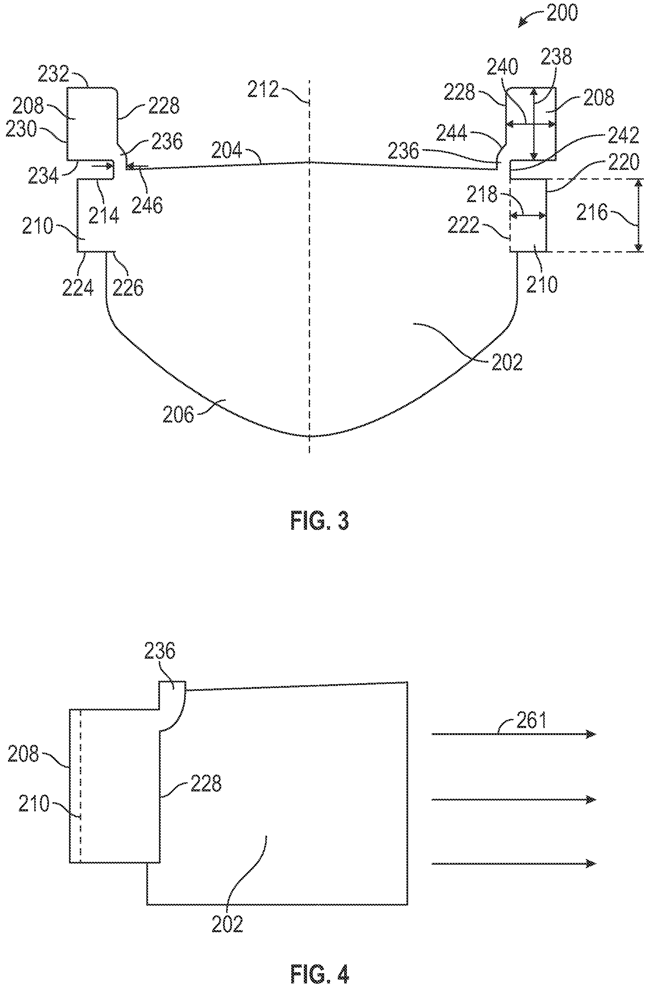

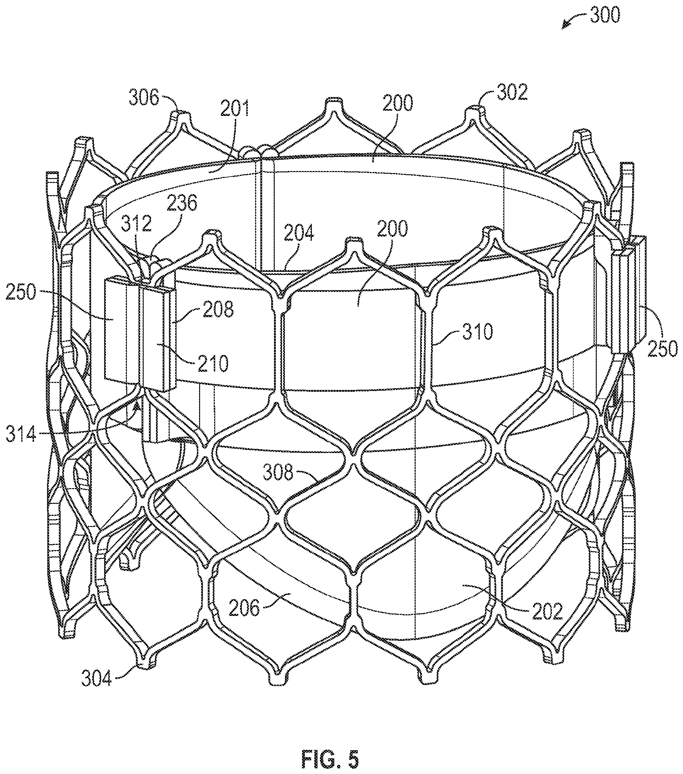

A leaflet 200 for a prosthetic heart valve, such as the prosthetic heart valve 10 of FIG. 1 or the prosthetic heart valve 300 shown in FIG. 5, is shown in a flattened configuration in FIG. 3. The leaflet 200 can be formed of pericardial tissue (e.g., bovine pericardial tissue), biocompatible synthetic materials, or various other suitable natural or synthetic materials as known in the art and described in U.S. Patent No. 6,730,118,

The leaflet 200 has a main body 202 with a free edge 204 (which can also be referred to as an outflow edge) and a cusp edge portion 206 (also referred to as an inflow edge portion). As described further below, the cusp edge portion 206 is configured to be attached to struts of a frame of a prosthetic heart valve and the free edge 204 is configured to move and contact respective free edges of the other leaflets of a leaflet assembly during closure of the leaflets (e.g., during diastole during operation of the prosthetic heart valve).

The leaflet 200 further comprises two sets of opposing commissure tabs disposed on opposite sides of the leaflet 200. For example, the leaflet 200 includes a pair of upper tabs 208 disposed on opposite sides of the leaflet 200 and a pair of lower tabs 210 disposed on opposite sides of the leaflet 200. The lower tabs 210 are disposed closer to the cusp edge portion 206 than the upper tabs 208.

For example, the cusp edge portion 206 terminates at its upper ends at the lower tabs 210. The lower tabs 210 extend laterally outward from the body 202 of the leaflet 200, relative to a central longitudinal axis 212 of the leaflet 200. As used herein, the axial direction can be a direction parallel to the central longitudinal axis 212 and the lateral direction can be perpendicular to the central longitudinal axis 212 (e.g., from one side of the leaflet to the opposite side of the leaflet, across the central longitudinal axis 212). As shown in FIG. 3, the central longitudinal axis 212 of the leaflet 200 extends between the inflow end and the outflow end of the leaflet 200.

An upper or outflow edge 214 of each lower tab 210 is positioned at an angle relative to the central longitudinal axis 212. In some examples, as shown in FIG. 3, the angle is 90 degrees.

In some examples, such as when the frame to which the leaflet 200 is to be attached is non-cylindrical (such as tapered, frustoconical, V-shaped, or Yshaped), the angle measured between the outflow edge 214 of each lower tab 210 and the central longitudinal axis 212 can be less than 90 degrees, such as 80 to 88 degrees.

In some examples, the angle measured between the outflow edge 214 of each lower tab 210 and the central longitudinal axis 212 can be selected based on a draft angle of the frame to which the leaflet 200 is to be attached, as described in PCT Publication No. WO 2022/026351,

Each lower tab 210 can have a height 216 and width 218. The width 218 can be measured between a laterally outer edge 220 and a laterally inner edge 222 (or integral or attached edge) of the corresponding lower tab 210. The inner edge 222 of the lower tab 210 can be aligned with a laterally inner edge 228 of the adjacent upper tab 208.

A slit 226 extends in the leaflet 200 laterally from an inflow edge 224 of the lower tab 210 to a point in the body 202 of the leaflet 200 that aligns with the inner edge 228 of the adjacent upper tab 208. The slit 226 allows for attachment of the corresponding upper tab 208 and the lower tab at a commissure, as described further below.

In some examples, as shown in FIG. 3, the outer edge 220 and inner edge 222 of each lower tab 210 are parallel to the central longitudinal axis 212. In some examples, as shown in FIG. 3, the inflow edge 224 and an outflow edge 214 of each lower tab are perpendicular to the central longitudinal axis 212.

Each upper tab 208 can have a substantially rectangular shape with the inner edge 228, a laterally outer edge 230 disposed opposite the inner edge 228, an outflow edge 232, and an inflow edge 234 disposed opposite the outflow edge 232. In some examples, the inner edge 228 and outer edge 230 can be referred to as side edges and are parallel to one another and the central longitudinal axis 212 (and thus they can be referred to as being vertical edges). In some examples, the outflow edge 232 and the inflow edge 234 are parallel to one another and disposed perpendicular to the inner edge 228 and outer edge 230.

In some examples, as shown in FIG. 3, the inner edge 228 and the outer edge 230 of each upper tab 208 are parallel to the outer edge 220 of the respective lower tab 210.

Each upper tab 208 has a height 238 and width 240. The width 240 of the upper tabs 208 is larger than the width 218 of the lower tabs 210. Thus, the outer edge 230 of each upper tab 208 extends farther laterally outward, away from the body 202 of the leaflet 200, than the outer edge 220 of the respective lower tab 210. As described further below, this makes assembly of the commissures to the frame easier and more accurate, thereby ensuring the valve can open as large as possible during operation of the prosthetic heart valve.

Each upper tab 208 is axially and laterally offset from the free edge 204 of the leaflet 200 by an offsetting portion 236 (which can also be referred to as a neck, neck portion, or connecting portion). The offsetting portion 236 extends between the lower tab 210 and the upper tab 208 on each side of the leaflet 200. For example, each offsetting portion 236 can include a relatively straight outer edge 242 that extends between the inflow edge 234 of the corresponding upper tab 208 and the outflow edge 214 of the corresponding lower tab 210.

Each offsetting portion 236 can also include a curved or arcuate inner edge 244 that curves between the inner edge 228 of the corresponding upper tab 208 and the free edge 204 of the leaflet 200. In some examples, the arcuate inner edge 244 curves 90 degrees between the inner edge 228 of the corresponding upper tab 208 and the free edge 204 of the leaflet 200.

The offsetting portions 236 have a relatively narrow width 246 which allows a length of the free edge 204 (measured between the two offsetting portions 236) to be as large as possible, thereby allowing the prosthetic valve to open wider and decrease pressure gradients across the prosthetic valve, during operation of the prosthetic valve (as described further below).

**In** some examples, the width 246 of the offsetting portions 236 can be less than half the width 218 of the lower tabs 210.

As described further below, each offsetting portion 236 offsets the inner edge 228 of the respective upper tab 208 laterally (or radially when attached to the frame) outward and away from the body 202 of the leaflet 200 when the upper tab 208 is folded over the lower tab 210, as shown in the schematic of FIG. 4. This reduces the likelihood of direct contact of the moving portion of the leaflet 200 (e.g., the body 202) with the inner edge 228 of the upper tabs 208, thereby increasing a durability of the leaflet 200.

A plurality of the leaflets 200 (e.g., three leaflets 200) can be assembled together into a leaflet assembly or valvular structure 201 and then secured to a frame of a prosthetic heart valve, such as the frame 302 of the prosthetic valve 300 shown in FIGS. 5-8. Though shown secured to frame 302, leaflets 200 can be used with a variety of prosthetic heart valve frames (such as frame 12 of FIG. 1).

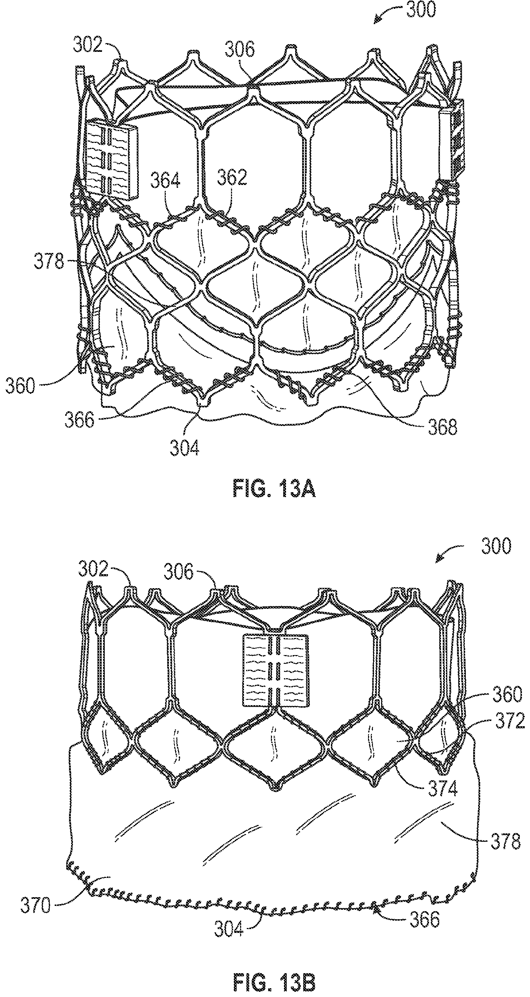

As shown in FIG. 5, the prosthetic valve 300 has an inflow end 304, an outflow end 306, and a valvular structure 201 comprising a plurality (e.g., three) leaflets 200 coupled to and supported by the frame 302. The prosthetic valve 300 can further comprise an inner and/or outer skirt, however, such components are omitted in FIG. 5 for purposes of illustration. An example of such inner and outer skirts secured to the frame 302 are shown in FIGS. 13A-15, as described below.

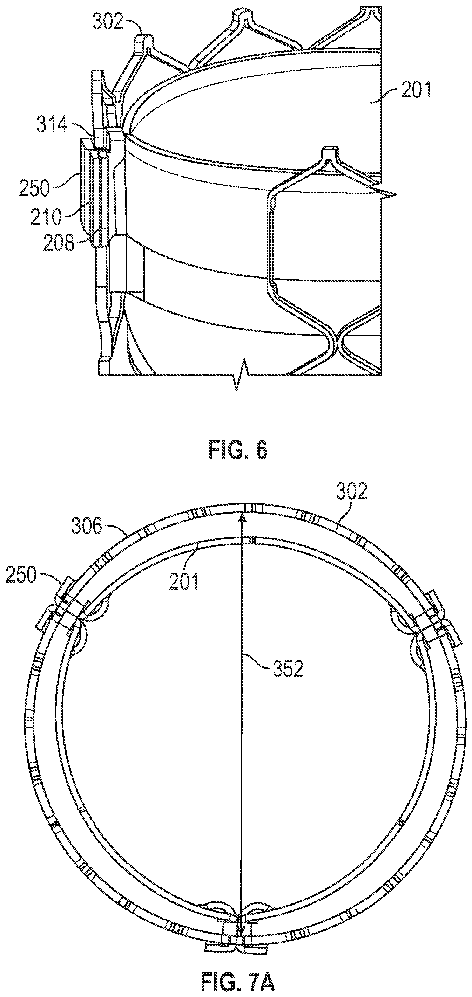

The frame 302 can comprise a plurality of interconnected struts 308 that are arranged into a plurality of rows of angled struts 308 disposed between the inflow end 304 and the outflow end 306 of the frame 302. The struts 308 define open cells 310 of the frame 302. The frame 302 has a plurality of circumferentially spaced slots, or commissure windows 312 that are adapted to mount commissures 250 of the valvular structure 201 to the frame 302. For example, the commissure windows 312 can be defined by axially extending window strut portions 314 of the frame 302, which can also be referred to herein as "commissure supports" (see FIG. 6 for a more detailed view of the commissure 250 with a portion of the struts of the frame removed to better visualize the folded tabs of the leaflet 200 forming the commissure 250). Each commissure window 312 is adapted to receive a pair of lower tabs 210 of a pair of adjacent leaflets 200 therethrough, as shown in FIGS. 5-8.

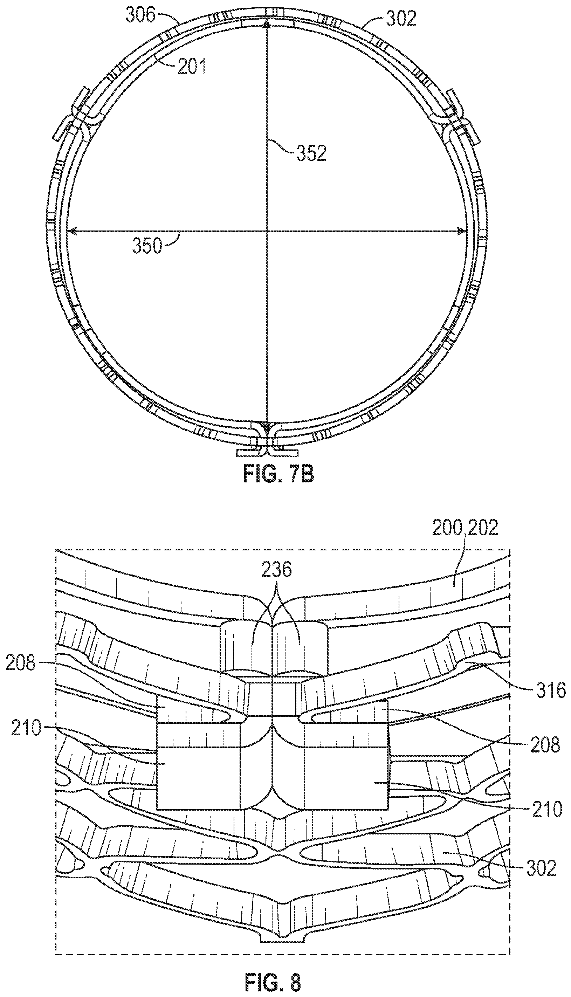

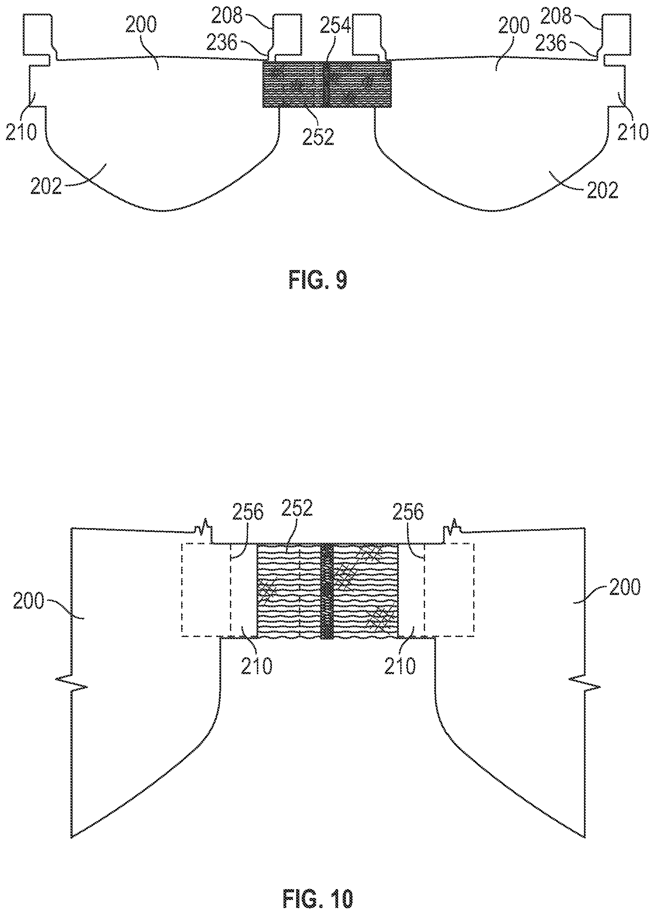

For example, referring to FIG. 8, adjacent lower tabs 210 of two adjacent leaflets 200 can be coupled together (e.g., via a post, flexible connector, or attachment member, as described further below with reference to FIGS. 9-12B), and the upper tabs 208 of the two adjacent leaflets 200 can be folded downward at their offsetting portions 236 such that the lower tabs 210 are disposed between the pair of upper tabs 208. The lower tabs 210 can then be inserted through a commissure window 312 in the frame 302 and folded across the radially outward facing surface 316 of the frame 302. Each lower tab 210 can be coupled to a respective upper tab 208 along a suture line. Further details on forming the commissures 250 using flexible connectors 252 is described below with reference to FIGS. 9-12B.

Further details on the frame 302 and other similar frames that can be used and assembled with the leaflets 200, as described herein, can be found in U.S. Patent No. 9,393, 110, and WO 2022/026351,

During typical valve operation, the leaflets 200 transition between a closed state in diastole, with their free edges 204 (outflow edges) coapting against each other, and an open state (see e.g., FIG. 7B) allowing blood to flow through the prosthetic valve 300. The outflow orifice through which the blood can flow determines the pressure gradient across the valve. Known valves can have valvular structures attached to the frame in such a manner that the outflow edges of each leaflet are spaced radially inward of the frame to prevent leaflet abrasion when the leaflets open under the flow of blood. In such valves, the effective outflow orifice (e.g., as determined by the position of the leaflets), also referred to as the geometric orifice area (GOA), can be narrower than the inflow orifice, producing a relatively high pressure gradient across the prosthetic valve. Accordingly, and particularly when small diameter valves are used, it is preferable to provide a large outflow orifice during systole to prevent elevated pressure gradients.

As shown in FIGS. 7A and 7B (where FIG. 7B is the fully open state of the valve), the valvular structure 201 of prosthetic valve 300 advantageously defines a relatively large GOA 350 (e.g., the leaflets 200 are near or touching an inner surface of the frame 302) when compared to the size of the outflow orifice 352 defined by the outflow end 306 of the frame 302. The term "GOA," as used herein, is defined as the open space through which blood can flow when the valvular structure 201 is in the open configuration. The GOA 350 of the outflow orifice 352 can be sized to provide a selected pressure gradient across the prosthetic valve 300. Such a configuration can be achieved by attaching the leaflets 200 to the frame 302 in such a manner that the radial distance between the outflow, free edges 204 of the leaflets 200 and the frame 302 (or the difference between the outflow orifice 352 and the GOA 350) is minimized.

The design of the leaflets 200, as described above with reference to FIG. 3, advantageously maximizes the GOA of the prosthetic valve 300 and reduces the pressure gradient across the prosthetic valve 300 compared to other, differently configured leaflets. In particular, the relatively narrower offsetting portions 236, the wider upper tabs 208 with an outer edge 230 that extends farther laterally outward than the outer edge 220 of the lower tabs 210, and the longer free edge 204 of the leaflets allow for the valvular structure 201 to open wider during valve operation (e.g., during systole), thereby decreasing the pressure gradient across the prosthetic valve 300.

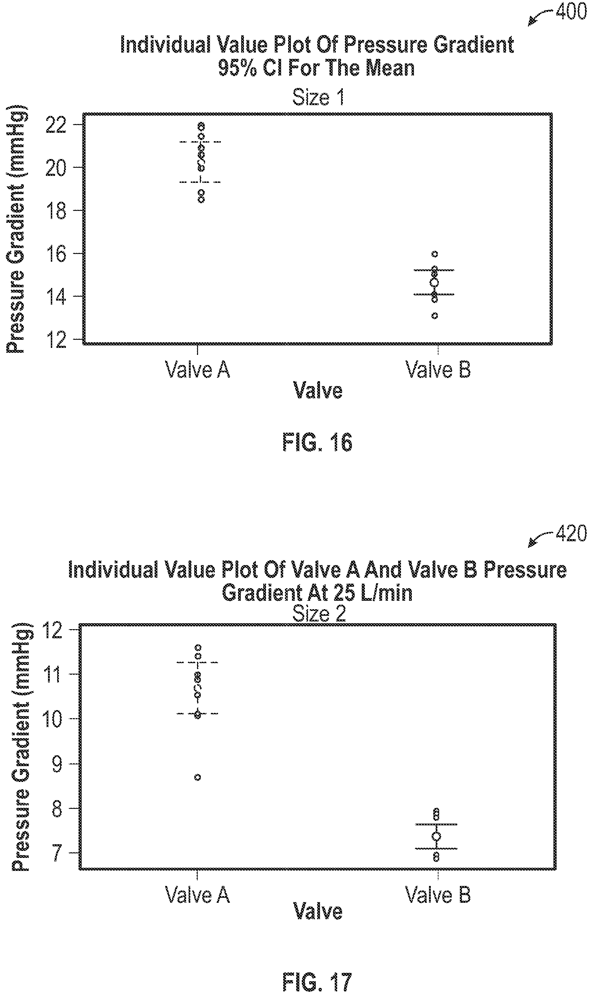

For example, as shown in the exemplary graphs 400 and 420 of FIGS. 16 and 17, respectively, Valve B which includes a valvular structure comprising the leaflets 200 (e.g., valve 300) has a lower pressure gradient across the valve than a different Valve A which includes a valvular structure comprising differently configured leaflets (e.g., leaflets without the geometrical advantages discussed above for leaflets 200) but are otherwise similarly constructed valves. In particular, the leaflets of Valve A do not include the wider upper tabs, the vertical inner and outer edges of the upper tabs, or the narrower offsetting portions that increase the length of the outflow edge of the leaflets, as described herein for leaflets 200.

In particular, graph 400 shows a plot of pressure gradient across the valve for Valve A and Valve B having a first size and graph 420 shows a plot of pressure gradient across the valve for Valve A and Valve B having a second size during steady forward flow through the valve (e.g., during operation of the valve). In graph 400, Valves A and B are 20-mm diameter valves and in graph 420, Valves A and B are 23-mm diameter valves.

As illustrated in the graphs, it can be seen that a prosthetic valve including the leaflets 200 (Valve B) experiences a substantially lower pressure gradient across the valve during forward flow through the valve than another valve that does not include the leaflets 200 (Valve A).

Additionally, the configuration of the upper tabs 208 and the offsetting portions 236 of the leaflet 200 can provide for increased durability, which in turn increases a longevity of the prosthetic valve 300. For example, during operation of the prosthetic valve 300, when its leaflets 200 transition into a closed state (for example, in diastole), an inwardly directed force is exerted on the commissures 250 to bend the leaflets 200 relative to the commissures 250 in an inwardly oriented direction. This is shown in the schematic of FIG. 4 which depicts an upper tab 208 folded over the respective lower tab 210, at the offsetting portion 236, and the inwardly (toward a central longitudinal axis of the prosthetic valve 300) directed force 261. The smoother, arcuate shape of the offsetting portion 236 can spread the stresses across the bending region between the upper tab 208 and lower tab 210, thereby reducing stress concentrations at the bending region and preserving a structural integrity of the leaflet 200 and commissure 250. Additionally, as mentioned above, the offsetting portions 236 offset the inner edge 228 of the upper tabs 208 away from the body 202 and moving portion of the leaflet 200. As a result, the durability of the leaflet 200 is further increased.

Turning now to FIGS. 9-13B, the assembly of the commissures 250 to the commissure windows 312 of the frame 302 is shown in more detail. As introduced above, the leaflets 200 can be secured to one another at their adjacent sides to form commissures 250 of the valvular structure 201. A plurality of flexible connectors 252 (one of which is shown in FIGS. 9-12B) can be used to interconnect pairs of adjacent sides of the leaflets 200 and to mount the leaflets 200 to the axially extending window strut portions 314 forming the commissure windows 312.

The flexible connectors 252 can be made from a piece of woven PET fabric, although other synthetic and/or natural materials can be used. Each flexible connector 252 can include a wedge 254 extending from the lower edge to the upper edge at the center of the flexible connector 252. The wedge 254 can comprise a non-metallic material, such as a rope or a piece of Ethibond 2-0 suture material, secured to the flexible connector 252 with a temporary suture. The wedge 254 helps prevent rotational movement of the leaflet tabs once they are secured to the axially extending window strut portions 314. In some examples, the connector 252 can have a series of inner and outer notches formed along its upper and lower edges that help with alignment with the leaflet tabs during assembly of the commissure 250.

FIGS. 9 and 10 show the adjacent sides of two leaflets 200 interconnected by a flexible connector 252 (FIG. 9 showing a first side view and FIG. 10 showing the opposite, second side view). The opposite end portions of the flexible connector 252 can be placed in an overlapping relationship with the lower tabs 210 with inner notches (which can be V-shaped notches in some examples or markers in some examples) aligned with the outer edges 220 of the lower tabs 210.

Each lower tab 210 can be secured to a corresponding end portion of the flexible connector 252 by suturing along a line extending from an outer notch or marker on the lower edge to an outer notch or marker on the upper edge of the connector 252, thereby forming stitching lines 256 (FIG. 10). Three leaflets 200 can be secured to each other side-to-side using three flexible connectors 252 to form the valvular structure 201.

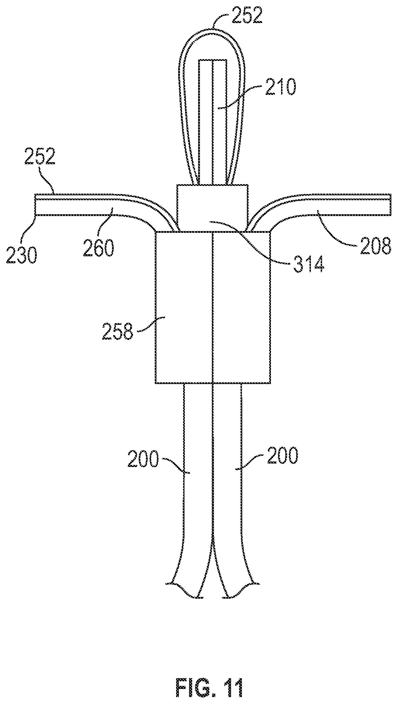

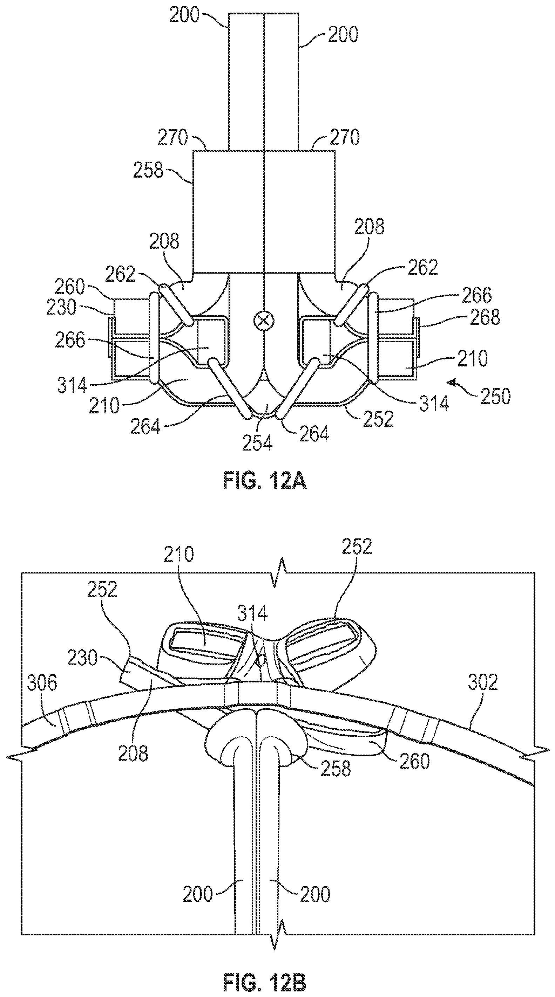

FIG. 12A is a cross-sectional view and FIG. 12B is a top view of a portion of the frame 302 and valvular structure 201 showing the adjacent commissure tabs of two leaflets 200 secured to corresponding axially extending window strut portions 314. FIG. 11 depicts an example approach for arranging the commissure tabs of the two adjacent leaflets 200 within the commissure window 312 forming by the axially extending window strut portions 314.

Prior to inserting the lower tabs 210 through the commissure window 312, the flexible connector 252 securing the two adjacent lower tabs 210 of the two adjacent leaflets 200 is folded widthwise (e.g., into the page in FIG. 9) and the upper tabs 208 are folded downwardly against the flexible connector 252 (over the lower tabs 210).

Each upper tab 208 is creased lengthwise (vertically) to assume an L-shape having an inner portion 258 folded against the inner surface of the leaflet 200 and an outer portion 260 folded against the connector 252 (FIGS. 11-12B). The outer portion 260 can then be sutured to the connector 252 along a suture line 262 (FIG. 12A). Next, the pair of lower tabs 210 connected by connector 252 is inserted through the commissure window 312 of corresponding axially extending window strut portions 314, as shown in FIG. 11.

The connector 252 and lower tabs 210 extending through the commissure window 312 can then be pressed radially inwardly at a center of the connector 252 (e.g., at the wedge 254) such that one of the lower tabs 210 and a portion of the connector 252 is folded against the frame 302 on one side of the axially extending window strut portions 314 and the other lower tab 210 and a portion of the connector 252 is folded against the frame 302 on other side of the axially extending window strut portions 314 (FIG. 12B).

A pair of suture lines 264 can be formed to retain the lower tabs 210 against the frame 302 in the manner shown in FIG. 12A. Each suture line 264 can extend through the connector 252, a lower tab 210, the wedge 254, and another portion of the connector 252. Then, as shown in FIG. 12A, each lower tab 210 is secured to a corresponding upper tab 208 with a primary suture line 266 that extends through one layer of the connector 252, the lower tab 210, another layer of the connector 252, another layer of the connector 252, and the upper tab 208.

As shown in FIGS. 11-12B, outer edges 230 of the upper tabs 208 are aligned with edges of the flexible connector 252 (with no gap in the lateral direction therebetween). This alignment is made possible by the larger width 240 of the upper tabs 208 (as described above with reference to FIGS. 3 and 4). Being able to align the edge of the flexible connector 252 with the outer edges 230 of the upper tabs 208 makes the assembly process easier and more accurate (e.g., reduces variability and increases consistency from valve to valve). As a result, a likelihood of all leaflets 200 of the valvular structure 201 being evenly arranged within the frame is increased, thereby ensuring the largest possible GOA.

In some examples, as shown in FIG. 12A, the suture material used to form the primary suture line 266 can be used to further form whip stitches 268 at the edges of the lower tabs 210 and upper tabs 208 that extend through two layers of connector 252 sandwiched between the upper and lower tabs 208, 210.

The leaflets 200 articulate primarily at inner edges 270 of the folded-down inner portions 258 in response to blood flowing through the valve during operation within the body, as opposed to articulating about the axial struts of the commissure windows 312.

In some examples, an inner skirt and an outer skirt (such as the inner skirt 16 and outer skirt 18 of prosthetic valve 10 of FIG. 1) can be secured to the frame 302 and form the prosthetic valve 300. For example, as shown in FIGS. 13A and 13B, after all three commissure 250 are secured to respective commissure windows 312, the cusp edge portions 206 of the leaflets 200 can be sutured to an inner skirt 360 (FIG. 13A). The inner skirt 360 can be attached to a fourth row of angled struts 362 of the frame 302, for example with a plurality of whip stitches 364. The inner skirt 360 is then further attached to a first row of angled struts 366 that define the inflow end 304 of the frame 302, for example with a plurality of whip stitches 368.

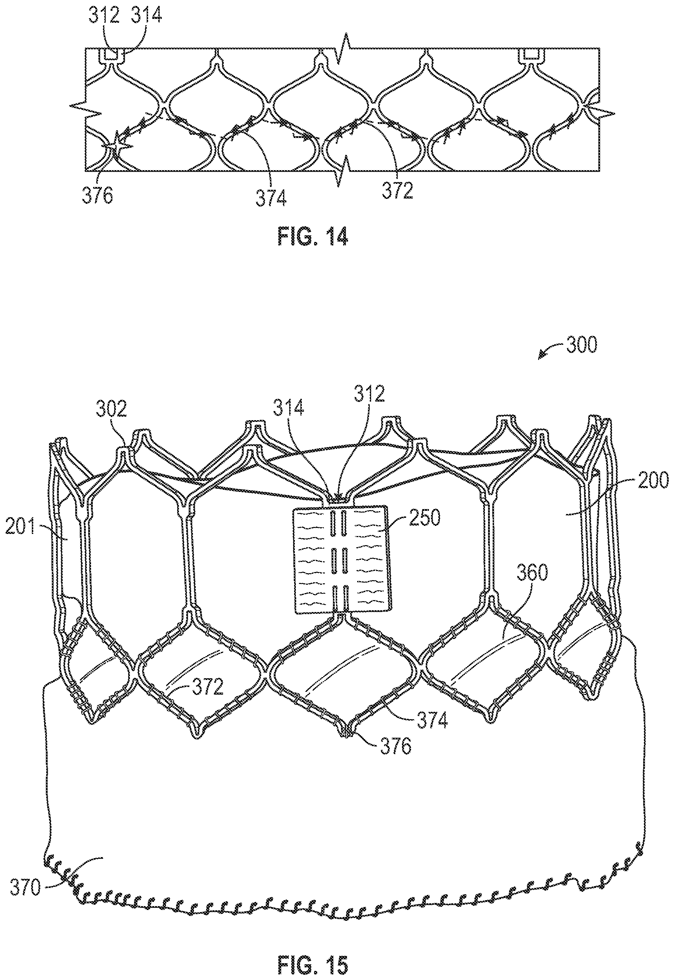

As shown in FIG. 13B, an outer skirt 370 is then positioned around the outer surface of the frame 302 and attached, along with the inner skirt 360, to a third row of angled struts 372 with a plurality of whip stitches 374 (shown schematically in FIG. 14 with arrows). In this way, the same stitch line comprising stitches 374 is used to attach both the outer skirt 370 and the inner skirt 360 to the third row of angled struts 372. In some examples, as shown in FIGS. 14 and 15, the line of whip stitches 374 has a single knot tail 376 that is formed around one of the angled struts of the third row of angled struts 372 that is disposed below a commissure window 312. By utilizing only a single knot tail 376 and by securing the outer skirt 370 and the inner skirt 360 to the third row of angled struts 372 using the same stitch line, an overall number of knot tails used to assemble the valve is minimized, thereby making the assembly process easier and minimizing the material interaction with the leaflets 200. For example, the likelihood that the single knot tail is pushed toward the leaflets 200 is reduced, thereby increasing a durability of the leaflets 200.

As shown in FIG. 13B, the outer skirt 370 can then be attached to a second row of angled struts 378 (e.g., with a plurality of whip stitches) and then the first row of angled struts 366 (e.g., with a plurality of whip stitches). The second row of angled struts 378 and first row of angled struts 366 are shown in FIG. 13A (with the outer skirt 370 removed) and indicated with arrows in FIG. 13B to denote their position underneath the outer skirt 370.

Additional details on the assembly of the leaflets 200 to the frame 302, or a similar prosthetic valve frame, can be found in U.S. Patent No. 9,393,110

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) is introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

## Claims

1. A leaflet (200) for a prosthetic valve (300), comprising:
a main body (202) with a free, outflow edge (204) and a cusp edge portion (206);
two lower tabs (210) disposed on opposite sides of the main body (202), wherein the cusp edge portion (206) terminates at upper ends thereof at the lower tabs (210), and the lower tabs (210) extend laterally outward from the main body (202) relative to a central longitudinal axis (212) of the leaflet (200);
two upper tabs (208) disposed on opposite sides of the main body (202) and extending laterally outward from the main body (202); and
two offsetting portions (236), each offsetting portion (236) extending between a respective lower tab (210) and upper tab (208) and offsetting the respective upper tab (208) axially and laterally away from the outflow edge (204) of the main body (202), wherein each upper tab (208) has opposing inner and outer edges (228, 230) that are parallel to one another and disposed parallel to the central longitudinal axis (212) of the leaflet (200).

2. The leaflet (200) of claim 1, wherein each lower tab (210) has an outer edge (220) that is laterally offset from the main body (202) and disposed parallel to the central longitudinal axis (212) of the leaflet (200) and the outer edge (230) of the respective upper tab (208).

3. The leaflet (200) of either claim 1 or claim 2, wherein the outer edge (230) of each upper tab (208) extends laterally outward farther than an outer edge (220) of a respective lower tab (210), relative to the central longitudinal axis (212) of the leaflet (200).

4. The leaflet (200) of any one of claims 1-3, wherein each upper tab (208) has an outflow edge (232) and an inflow edge (234) disposed opposite the outflow edge (232), wherein the inflow edge (234) is disposed closer to a respective lower tab (210) than the outflow edge (232).

5. The leaflet (200) of claim 4, wherein the inflow and outflow edges (234, 232) are perpendicular to the inner and outer edges (228, 230) of the upper tab (208).

6. The leaflet (200) of any one of claims 1-5, wherein each offsetting portion (236) has an outer edge that extends between an inflow edge (234) of the respective upper tab (208) and an outflow edge (214) of the respective lower tab (210) and an arcuate inner edge (244) that curves between the inner edge (228) of the respective upper tab (208) and the outflow edge (204) of the main body (202).

7. The leaflet (200) of claim 6, wherein the arcuate inner edge (244) curves 90 degrees between the inner edge (228) of the respective upper tab (208) and the outflow edge (204) of the main body (202).

8. The leaflet (200) of either claim 6 or claim 7, wherein the outer edge (242) of each offsetting portion (236) extends parallel to the central longitudinal axis (212) of the leaflet (200).

9. The leaflet (200) of any one of claims 1-8, wherein a width (246) of each offsetting portion (236) is less than half a width (218) of each lower tab (210), and wherein the outflow edge (204) of the main body (202) extends between the two offsetting portions (236).

10. The leaflet (200) of any one of claims 1-9, wherein each upper tab (208) has an inner edge (228) disposed opposite the outer edge (230), and wherein the inner and outer edges (228, 230) of each upper tab (208) are parallel to the central longitudinal axis (212) of the leaflet (200).

11. The leaflet (200) of any one of claims 1-10, wherein each upper tab (208) has opposing inflow and outflow edges (234, 232) that are perpendicular to the central longitudinal axis (212) of the leaflet (200).

12. The leaflet (200) of any one of claims 1-11, wherein each offsetting portion (236) has a first width defined between its inner and outer edges (244, 242) that is less than half a second width of the lower tabs (210).

13. The leaflet (200) of any one of claims 1-12, wherein a slit (226) extends in the leaflet (200) laterally from the inflow edge (224) of the lower tab (210) to a point in the main body (202) of the leaflet (200) that aligns with the inner edge (228) of the adjacent upper tab (208).

14. A prosthetic heart valve (300), comprising a plurality of the leaflets (200) of any one of claims 1-13, wherein for each leaflet (200), the upper tabs (208) are folded over the respective lower tabs (210).

15. The prosthetic heart valve (300) of claim 14, wherein the cusp edge portion (206) of each leaflet (200) is attached to struts of a frame (302) of the prosthetic heart valve (300) via an inner skirt (16) of the prosthetic heart valve (300), and wherein the outflow edge (204) of the main body (202) of each leaflet (200) is free to move during operation of the prosthetic heart valve (300) to regulate a flow of blood through the prosthetic heart valve (300).

## Patentansprüche

1. Klappensegel (200) für eine Klappenprothese (300), umfassend:
einen Hauptkörper (202) mit einem freien Ausströmrand (204) und einem Kuspenrandabschnitt (206);
zwei untere Laschen (210), die an gegenüberliegenden Seiten des Hauptkörpers (202) angeordnet sind, wobei der Kuspenrandabschnitt (206) an oberen Enden davon an den unteren Laschen (210) endet, und die unteren Laschen (210) sich von dem Hauptkörper (202) lateral auswärts relativ zu einer zentralen Längsachse (212) des Klappensegels (200) erstrecken;
zwei obere Laschen (208), die an gegenüberliegenden Seiten des Hauptkörpers (202) angeordnet sind und sich von dem Hauptkörper (202) lateral auswärts erstrecken; und
zwei Versatzabschnitte (236), wobei jeder Versatzabschnitt (236) sich zwischen einer entsprechenden unteren Lasche (210) und oberen Lasche (208) erstreckt und die entsprechende obere Lasche (208) axial und lateral von dem Ausströmrand (204) des Hauptkörpers (202) weg versetzt, wobei jede obere Lasche (208) gegenüberliegende innere und äußere Ränder (228, 230) aufweist, die parallel zueinander sind und parallel zu der zentralen Längsachse (212) des Klappensegels (200) angeordnet sind.

2. Klappensegel (200) nach Anspruch 1, wobei jede untere Lasche (210) einen äußeren Rand (220) aufweist, der von dem Hauptkörper (202) lateral versetzt ist und parallel zu der zentralen Längsachse (212) des Klappensegels (200) und dem äußeren Rand (230) der entsprechenden oberen Lasche (208) angeordnet ist.

3. Klappensegel (200) nach Anspruch 1 oder Anspruch 2, wobei der äußere Rand (230) von jeder oberen Lasche (208) sich weiter als ein äußerer Rand (220) der entsprechenden unteren Lasche (210) relativ zu der zentralen Längsachse (212) des Klappensegels (200) lateral auswärts erstreckt.

4. Klappensegel (200) nach einem der Ansprüche 1 bis 3, wobei jede obere Lasche (208) einen Ausströmrand (232) und einen Einströmrand (234) aufweist, der gegenüber von dem Ausströmrand (232) angeordnet ist, wobei der Einströmrand (234) näher an einer entsprechenden unteren Lasche (210) als der Ausströmrand (232) angeordnet ist.

5. Klappensegel (200) nach Anspruch 4, wobei der Einströmrand und der Ausströmrand (234, 232) rechtwinklig zu dem inneren Rand und dem äußeren Rand (228, 230) der oberen Lasche (208) angeordnet sind.

6. Klappensegel (200) nach einem der Ansprüche 1 bis 5, wobei jeder Versatzabschnitt (236) einen äußeren Rand, der sich zwischen einem Einströmrand (234) der entsprechenden oberen Lasche (208) und einem Ausströmrand (214) der entsprechenden unteren Lasche (210) erstreckt, und einen bogenförmigen inneren Rand (244) aufweist, der sich zwischen dem inneren Rand (228) der entsprechenden oberen Lasche (208) und dem Ausströmrand (204) des Hauptkörpers (202) krümmt.

7. Klappensegel (200) nach Anspruch 6, wobei sich der bogenförmige innere Rand (244) zwischen dem inneren Rand (228) der entsprechenden oberen Lasche (208) und dem Ausströmrand (204) des Hauptkörpers (202) um 90 Grad krümmt.

8. Klappensegel (200) nach Anspruch 6 oder Anspruch 7, wobei der äußere Rand (242) von jedem Versatzabschnitt (236) sich parallel zu der zentralen Längsachse (212) des Klappensegels (200) erstreckt.

9. Klappensegel (200) nach einem der Ansprüche 1 bis 8, wobei eine Breite (246) von jedem Versatzabschnitt (236) kleiner als eine halbe Breite (218) von jeder unteren Lasche (210) ist, und wobei der Ausströmrand (204) des Hauptkörpers (202) sich zwischen den zwei Versatzabschnitten (236) erstreckt.

10. Klappensegel (200) nach einem der Ansprüche 1 bis 9, wobei jede obere Lasche (208) einen inneren Rand (228) aufweist, der gegenüber von dem äußeren Rand (230) angeordnet ist, und wobei der innere Rand und der äußere Rand (228, 230) von jeder oberen Lasche (208) parallel zu der zentralen Längsachse (212) des Klappensegels (200) sind.

11. Klappensegel (200) nach einem der Ansprüche 1 bis 10, wobei jede obere Lasche (208) gegenüberliegende Einström- und Ausströmränder (234, 232) aufweist, die rechtwinklig zu der zentralen Längsachse (212) des Klappensegels (200) sind.

12. Klappensegel (200) nach einem der Ansprüche 1 bis 11, wobei jeder Versatzabschnitt (236) eine erste Breite aufweist, die zwischen seinem inneren Rand und seinem äußeren Rand (244, 242) definiert ist, die kleiner als eine zweite Breite der unteren Laschen (210) ist.

13. Klappensegel (200) nach einem der Ansprüche 1 bis 12, wobei sich ein Schlitz (226) in dem Klappensegel (200) lateral von dem Einströmrand (224) der unteren Lasche (210) zu einem Punkt in dem Hauptkörper (202) des Klappensegels (200) erstreckt, der mit dem inneren Rand (228) der benachbarten oberen Lasche (208) ausgerichtet ist.

14. Prothetische Herzklappe (300), umfassend eine Vielzahl der Klappensegel (200) nach einem der Ansprüche 1 bis 13, wobei für jedes Klappensegel (200) die oberen Laschen (208) über den entsprechenden unteren Laschen (210) gefaltet sind.

15. Prothetische Herzklappe (300) nach Anspruch 14, wobei der Kuspenrandabschnitt (206) von jedem Klappensegel (200) an Streben eines Rahmens (302) der prothetischen Herzklappe (300) mittels einer inneren Schürze (16) der prothetischen Herzklappe (300) angebracht ist, und wobei der Ausströmrand (204) des Hauptkörpers (202) jedes Klappensegels (200) sich während des Betriebs der prothetischen Herzklappe (300) frei bewegen kann, um einen Blutfluss durch die prothetische Herzklappe (300) hindurch zu regulieren.

## Revendications

1. Feuillet (200) pour une valvule prothétique (300), comprenant :
un corps principal (202) pourvu d'un bord de sortie (204) libre et d'une partie de bord de cuspide (206) ;
deux languettes inférieures (210) disposées sur des côtés opposés du corps principal (202), la partie de bord de cuspide (206) se terminant en ses extrémités supérieures au niveau des languettes inférieures (210) et les languettes inférieures (210) s'étendant latéralement vers l'extérieur à partir du corps principal (202) par rapport à un axe longitudinal central (212) du feuillet (200) ;
deux languettes supérieures (208) disposées sur des côtés opposés du corps principal (202) et s'étendant latéralement vers l'extérieur à partir du corps principal (202) ; et
deux parties de décalage (236), chaque partie de décalage (236) s'étendant entre une languette inférieure (210) respective et une languette supérieure (208) et décalant la languette supérieure (208) respective axialement et latéralement à l'opposé du bord de sortie (204) du corps principal (202), chaque languette supérieure (208) présentant des bords interne et externe (228, 230) opposés qui sont parallèles l'un à l'autre et disposés parallèlement à l'axe longitudinal central (212) du feuillet (200).

2. Feuillet (200) selon la revendication 1, chaque languette inférieure (210) présentant un bord externe (220) qui est décalé latéralement par rapport au corps principal (202) et disposé parallèlement à l'axe longitudinal central (212) du feuillet (200) et au bord externe (230) de la languette supérieure (208) respective.

3. Feuillet (200) selon soit la revendication 1 soit la revendication 2, le bord externe (230) de chaque languette supérieure (208) s'étendant latéralement vers l'extérieur plus loin qu'un bord externe (220) d'une languette inférieure (210) respective, par rapport à l'axe longitudinal central (212) du feuillet (200).

4. Feuillet (200) selon l'une quelconque des revendications 1 à 3, chaque languette supérieure (208) présentant un bord de sortie (232) et un bord d'entrée (234) disposé à l'opposé du bord de sortie (232), le bord d'entrée (234) étant disposé plus près d'une languette inférieure (210) respective que le bord de sortie (232).

5. Feuillet (200) selon la revendication 4, les bords d'entrée et de sortie (234, 232) étant perpendiculaires aux bords interne et externe (228, 230) de la languette supérieure (208).

6. Feuillet (200) selon l'une quelconque des revendications 1 à 5, chaque partie de décalage (236) présentant un bord externe qui s'étend entre un bord d'entrée (234) de la languette supérieure (208) respective et un bord de sortie (214) de la languette inférieure (210) respective et un bord interne arqué (244) qui s'incurve entre le bord interne (228) de la languette supérieure (208) respective et le bord de sortie (204) du corps principal (202).

7. Feuillet (200) selon la revendication 6, le bord interne arqué (244) s'incurvant à 90 degrés entre le bord interne (228) de la languette supérieure (208) respective et le bord de sortie (204) du corps principal (202).

8. Feuillet (200) selon soit la revendication 6 soit la revendication 7, le bord externe (242) de chaque partie de décalage (236) s'étendant parallèlement à l'axe longitudinal central (212) du feuillet (200).

9. Feuillet (200) selon l'une quelconque des revendications 1 à 8, une largeur (246) de chaque partie de décalage (236) étant inférieure à la moitié d'une largeur(218) de chaque languette inférieure (210) et le bord de sortie (204) du corps principal (202) s'étendant entre les deux parties de décalage (236).

10. Feuillet (200) selon l'une quelconque des revendications 1 à 9, chaque languette supérieure (208) présentant un bord interne (228) disposé à l'opposé du bord externe (230) et les bords interne et externe (228, 230) de chaque languette supérieure (208) étant parallèles à l'axe longitudinal central (212) du feuillet (200).

11. Feuillet (200) selon l'une quelconque des revendications 1 à 10, chaque languette supérieure (208) présentant des bords d'entrée et de sortie (234, 232) opposés qui sont perpendiculaires à l'axe longitudinal central (212) du feuillet (200).

12. Feuillet (200) selon l'une quelconque des revendications 1 à 11, chaque partie de décalage (236) présentant une première largeur définie entre ses bords interne et externe (244, 242) qui est inférieure à la moitié d'une seconde largeur des languettes inférieures (210).

13. Feuillet (200) selon l'une quelconque des revendications 1 à 12, une encoche (226) s'étendant dans le feuillet (200) latéralement depuis le bord d'entrée (224) de la languette inférieure (210) jusqu'à un point dans le corps principal (202) du feuillet (200) qui s'aligne sur le bord interne (228) de la languette supérieure adjacente (208).

14. Valvule cardiaque prothétique (300), comprenant une pluralité de feuillets (200) selon l'une quelconque des revendications 1 à 13, dans laquelle, pour chaque feuillet (200), les languettes supérieures (208) sont pliées sur les languettes inférieures respectives (210).

15. Valvule cardiaque prothétique (300) selon la revendication 14, la partie de bord de cuspide (206) de chaque feuillet (200) étant fixée à des entretoises d'un cadre (302) de la valvule cardiaque prothétique (300) par l'intermédiaire d'une collerette interne (16) de la valvule cardiaque prothétique (300), et le bord de sortie (204) du corps principal (202) de chaque feuillet (200) étant libre de se déplacer pendant le fonctionnement de la valvule cardiaque prothétique (300) afin de réguler un écoulement de sang à travers la valvule cardiaque prothétique (300).
